# EUROPEAN PATENT SPECIFICATION

(11) **EP 0 607 122 B1**
(45) Date of publication and mention of the grant of the patent: **18.07.2001**
(21) Application number: 91908740.3
(22) Date of filing: 22.04.1991
(51) Int. Cl.: A61K 31/43, A61K 31/42

(54) **VETERINARY TREATMENT**
TIERÄRZTLICHE BEHANDLUNG
TRAITEMENT VETERINAIRE

(30) Priority: 26.04.1990 GB 9009446
(43) Date of publication of application: 27.07.1994
(73) Proprietor: PFIZER INC., New York, N.Y. 10017-5755 (US)
(72) Inventor: CRACKNELL, Victor, Charles SmithKline Beecham,, Surrey KT20 7NT (GB)
(74) Representative: Wilkinson, Stephen John
(86) International application number: GB9100635
(87) International publication number: WO9116050

(56) References cited:
- EP-A- 0 010 904
- US-A- 1 508 977
- US-A- 4 525 352
- US-A- 4 529 720
- Unlisted Drugs, vol. 37, no. 1, January 1985, (Chatham, New Jersey, US), see page 13
- Unlisted Drugs, vol. 38, no.6, June 1986, (Chatham, New Jersey, US),
- PRESCOTT, J. F. et al. ANTIMICROBIAL THERAPY IN VETERINARY MEDICINE 2nd Edition IOWA STATE UNIVERSITY PRESS
- : "THE MERCK INDEX", , TWELFTH EDITION, PAGES 625-626, 1611, WHITEHOUSE STATION, NJ

## Description

This invention relates to a method for the treatment of bacterial infections in pigs, particularly those infections associated with farrowing fever and bacterial pneumonia.

GB-B-2 005 538 describes a dry pharmaceutical composition, which comprises 20mg to 1500mg of amoxycillin trihydrate, 20mg to 500mg of potassium clavulanate and a pharmaceutically acceptable carrier, with the proviso that the weight ratio of amoxycillin trihydrate to potassium clavulanate is from 6:1 to 1:1.

Unlisted Drugs, vol. 37, no. 1, January 1985, page 13, abstract e describes Synulox^{R}, an oral bolus containing amoxycillin and clavulanic acid which may be used for the treatment of enteritis and navel ill in calves.

Unlisted Drugs, vol. 38, no. 6, June 1986 mentions Clavamox^{R}, a veterinary tablet containing amoxycillin trihydrate and potassium clavulanate, which is an antibacterial for dogs and cats.

EP-A-0010904 discloses intramammary compositions containing amoxycillin, a salt of clavulanic acid and molecular sieve powder. The compositions are administered directly into the mammary gland.

According to the present invention there is provided the use of a formulation comprising an effective amount of amoxycillin or a veterinarily acceptable derivative thereof, clavulanic acid or a veterinarily acceptable derivative thereof, and a veterinarily acceptable carrier in the manufacture of a medicament for use by intramuscular injection in the treatment of farrowing fever and bacterial pneumonia in pigs.

The formulation has notable bactericidal activity against the bacteria associated with the diseases farrowing fever and bacterial pneumonia in particular

### Actinobacillus pleuropneumoniae.

Suitably the clavulanic acid used in the formulation is in the form of a veterinarily acceptable salt such as potassium clavulanate.

Suitably the amoxycillin used in the formulation is in the form of the trihydrate or a veterinarily acceptable ester or salt of amoxycillin such as the sodium salt.

The preferred weight ratio of amoxycillin or derivative to clavulanic acid or derivative is from 6:1 to 1:1.

Advantageously, a liquid formulation of the invention comprises 36mg/ml clavulanic acid (as potassium clavulanate) and 140mg/ml amoxycillin (as amoxycillin trihydrate).

Preferred veterinarily acceptable carriers include for example veterinarily acceptable oils such as mineral oils or fractionated coconut oil such as Miglyol 840.

The formulation is administered to the animal by intramuscular injection.

The formulation is typically prepared as an off-white suspension and is presented as a 'Ready-to-Use' form, that is the formulation is prepared and packed in a suitable container, wherein it is ready for administration to the animal in need thereof.

The dosage rate will vary according to the size of the animal. A suitable dosage rate is generally between 2 and 25mg/kg bodyweight of the animal, for example about 8.75mg/kg.

Typically more than a single dose of the formulation will be required for the treatment of the bacterial infections, suitably one dose per day for 3 to 5 days is required. In cases of severe infections prolonged treatment may be required.

Veterinary formulations for use in the present invention may be prepared by mixing the ingredients thereof in the required proportions.

The formulation is then packaged into an appropriate container ready for administration.

The following Example illustrates the invention.

| Formulation | g/100ml |
|---|---|
| amoxycillin trihydrate | 14.0 |
| potassium clavulanate | 3.6 |
| phenol | 0.5 |
| Miglyol 840 | to 100ml |

### Clinical data

### CASE STUDY 1

Naturally-occuring cases of farrowing fever in pigs were diagnosed on clinical grounds, then treated on an alternating basis with either Synulox^{*} or a control drug. The control drug was a penicillin G/streptomycin mixture. Treatment was administered once daily for a minimum of 3 days. Synulox^{*} was given at a dose rate of lml per 20kg of body weight.

The clinical response to treatment was scored on a pre-defined scale:
CRS 1 = excellent
CRS 2 = food
CRS 3 = fair
CRS 4 = poor
(CRS = clinical response score)

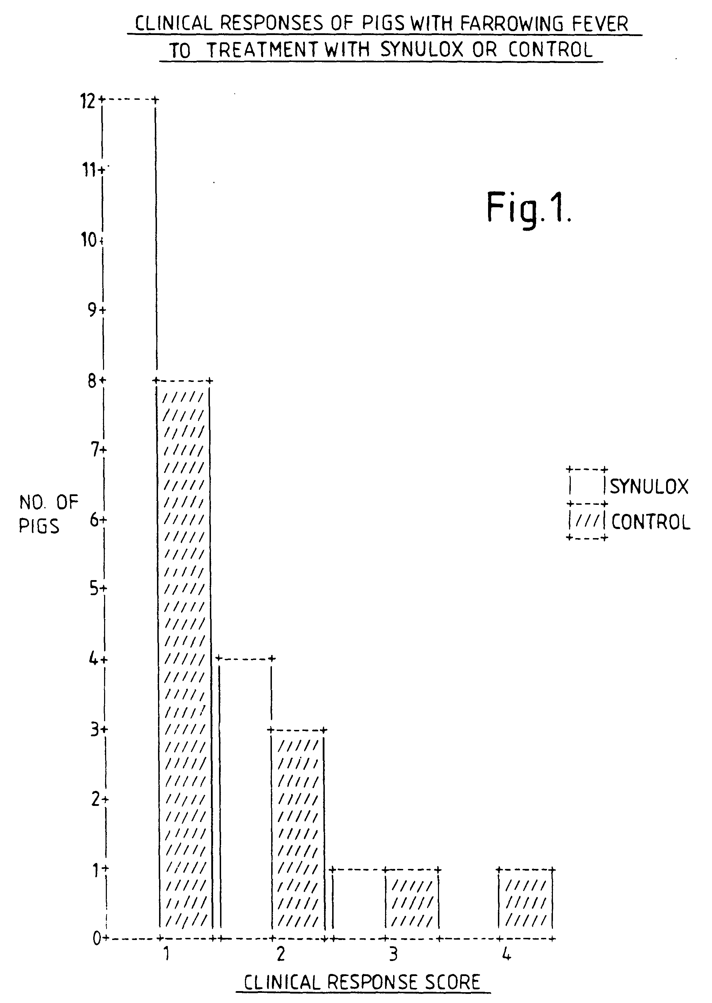

From the results which are summarised in FIG 1, it can be seen that Synulox^{*} has good efficacy in the treatment of farrowing fever in pigs.

### CASE STUDY 2

A total of 229 pigs with pneumonia caused by Actinobacillus (Haemophilus) pleuropneumonia were involved in this study.

156 pigs were treated with Synulox* and 73 were treated with a control drug, oxytetracycline (1ml/kg body weight administered intramuscularly). Synulox* was injected intramuscularly at a dose rate of lml/20kg body weight. Treatment was continued daily until clinical signs of cough, dyspnoea, anorexia and depression were remitted and clinical responses were stored on the basis of the number of treatments required to achieve remissision. Clinical response was scored as follows:
CRS 1 = excellent
CRS 2 = good
CRS 3 = fair
CRS 4 = poor

* Synulox is a Trademark of Beecham Group p.l.c.

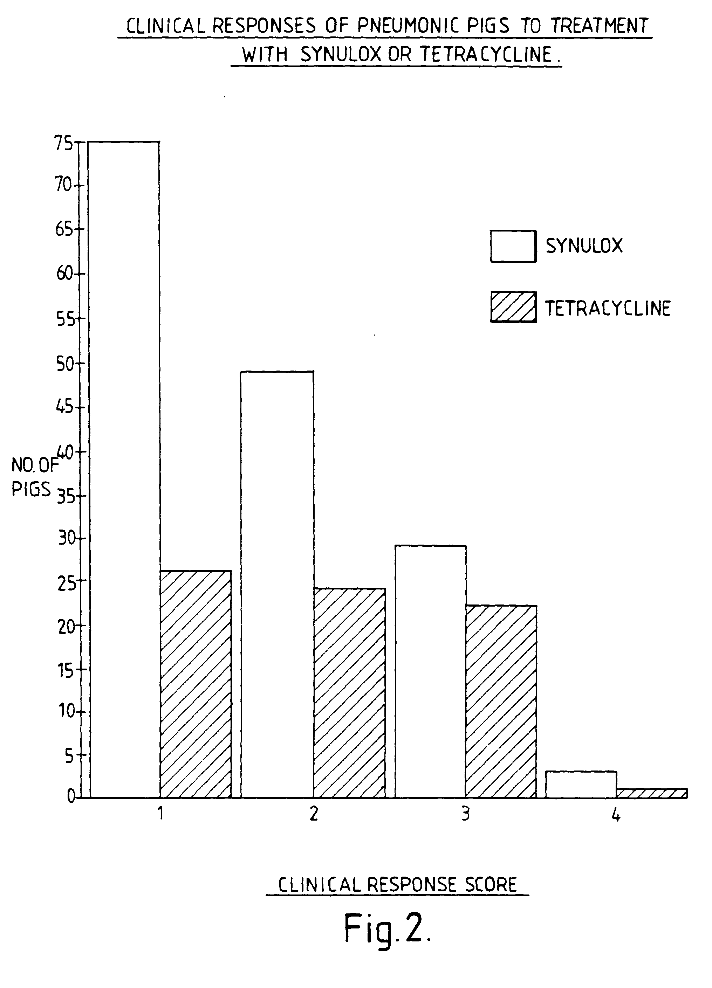

The clinical response scores are summarised in FIG 2, wherein it was shown that of the total of 156 pigs treated with Synulox* 48.1% showed an ''excellent'' response, 31.4% had a ''good'' response, 18.6% ''fair'' and only 1.9% "poor" against 35.6% of pigs treated with the control showing an ''excellent'' response 32.9%, ''good'', 31.5% ''fair'' and 0% ''poor''. It was therefore concluded that Synulox* has good efficacy in the treatment of bacterial pneumonia in pigs.

Synulox is an oily suspension contining 35mg/ml clavulanic acid (K salt) and 140mg/ml amoxycillin (trihydrate).

## Claims

1. Use of a formulation comprising an effective amount of amoxycillin or a veterinarily acceptable derivative thereof, clavulanic acid or a veterinarily acceptable derivative thereof, and a veterinarily acceptable carrier, in the manufacture of a medicament for use by intramuscular injection in the treatment of farrowing fever or bacterial pneumonia in pigs.

2. The use according to claim 1, wherein the clavulanic acid is used in the form of potassium clavulanate.

3. The use according to claim 1 or 2, wherein the amoxycillin is used in the form of the trihydrate or the sodium salt.

4. The use according to claim 1, 2, or 3, wherein the weight ratio of amoxycillin or derivative to clavulanic acid or derivative is from 6:1 to 1:1.

5. The use according to claim 1, 2, 3, or 4, wherein the veterinarily acceptable carrier is an oil suitable for injection.

## Patentansprüche

1. Verwendung einer Formulierung, umfassend eine wirksame Menge von Amoxycillin oder einem veterinär verträglichen Derivat davon, Clavulansäure oder einem veterinär verträglichen Derivat davon, und einen veterinär verträglichen Träger bei der Herstellung eines Arzneimittels zur Verwendung bei der Behandlung von Wurffieber oder bakterieller Pneumonie bei Schweinen, durch intramuskuläre Injektion.

2. Verwendung nach Anspruch 1, wobei die Clavulansäure in Form von Kaliumclavulanat verwendet wird.

3. Verwendung nach Anspruch 1 oder 2, wobei das Amoxycillin in Form des Trihydrats oder des Natriumsalzes verwendet wird.

4. Verwendung nach Anspruch 1, 2 oder 3, wobei das Gewichtsverhältnis von Amoxycillin oder einem Derivat zu Clavulansäure oder einem Derivat 6:1 bis 1:1 ist.

5. Verwendung nach Anspruch 1, 2, 3 oder 4, wobei der veterinär verträgliche Träger ein zur Injektion geeignetes öl ist.

## Revendications

1. Utilisation d'une formulation comprenant une quantité efficace d'amoxicilline ou d'un de ses dérivés acceptables du point de vue vétérinaire, d'acide clavulanique ou d'un de ses dérivés acceptables du point de vue vétérinaire, et un support acceptable du point de vue vétérinaire, dans la production d'un médicament destiné à être utilisé par injection intramusculaire dans le traitement de la fièvre de la mise bas ou de la pneumonie bactérienne chez les porcs.

2. Utilisation suivant la revendication 1, dans laquelle l'acide clavulanique est utilisé sous forme de clavulanate de potassium.

3. Utilisation suivant la revendication 1 ou 2, dans laquelle l'amoxicilline est utilisée sous forme du trihydrate ou du sel de sodium.

4. Utilisation suivant la revendication 1, 2 ou 3, dans laquelle le rapport pondéral de l'amoxicilline ou dérivé à l'acide clavulanique ou dérivé est compris dans l'intervalle de 6:1 à 1:1.

5. Utilisation suivant la revendication 1, 2, 3 ou 4, dans laquelle le support acceptable du point de vue vétérinaire est une huile convenable à des fins d'injection.
